# EUROPÄISCHE PATENTSCHRIFT

(11) **EP 1 072 321 B1**
(45) Veröffentlichungstag und Bekanntmachung des Hinweises auf die Patenterteilung: **25.01.2006**
(21) Anmeldenummer: 00109846.6
(22) Anmeldetag: 10.05.2000
(51) Int. Cl.: B05B 13/06, B05C 7/08, A61M 5/31

(54) **Verfahren zur Beschichtung von Primärpackmitteln und Vorrichtung zur Durchführung des Verfahrens**
Coating process for containers and device for carrying out said process
Processus de revêtement d'emballages et dispositif pour l'exécution d'un tel processus

(30) Priorität: 24.07.1999 DE 19934841
(43) Veröffentlichungstag der Anmeldung: 31.01.2001
(73) Patentinhaber: Arzneimittel GmbH Apotheker Vetter & Co. Ravensburg, D-88212 Ravensburg (DE)
(72) Erfinder: Vetter, Udo J., 88214 Ravensburg (DE)
(74) Vertreter: Dziewior, Joachim

(56) Entgegenhaltungen:
- EP-A- 0 962 229
- EP-B- 0 675 315
- DE-A- 3 045 950
- US-A- 3 335 700
- US-A- 5 338 312

## Beschreibung

Die Erfindung betrifft ein Verfahren zur Beschichtung von Primärpackmitteln, insbesondere zur innenseitigen Beschichtung von kleinvolumigen Spritzenzylindern, mit einem Silikonfilm, bei welchem ein ßeschichtungsring mit Silikonöl benetzt wird, daß sodann der Beschichtungsring in den Spritzenzylinder eingebracht und in axialer Richtung an der Innenwand entlang geführt wird, wobei der Beschichtungsring der Innenwand vollständig anliegt, und daß schließlich beim Zurückziehen des Beschichtungsrings ein in Bewegungsrichtung dahinter angeordneter Abstreifring das überschüssige Silikonöl unter Bildung eines homogenen, gleichmäßig dicken Silikonfilms abträgt. Weiter betrifft die Erfindung eine Vorrichtung zur Durchführung dieses Verfahrens.

Bei vorgefüllten Spritzen- und Karpulensystemen werden zur Abdichtung des pharmazeutischen Wirkstoffes Gummistopfen verwendet. Die Spritzen- bzw. Karpulenzylinder bestehen dabei üblicherweise aus Glas der hydrolytischen Klasse 1. Bei der Injektion werden die im Spritzen- bzw. Karpulenzylinder angeordneten Gummistopfen über eine Kolbenstange zum kanülenseitigen Ende hin gedrückt.

Bei dieser medizinischen Applikation des Wirkstoffes muß sich der Gummistopfen im Glaskörper leicht verschieben lassen. Um diese geringen Verschiebekräfte zu erreichen, müssen sowohl der Gummistopfen wie auch die Innenseite des Glaszylinders jeweils mit Silikonöl beschichtet werden.

Hierbei ist es einerseits erforderlich, das Silikonöl homogen auf die Innenfläche des Glaskörpers aufzutragen und eine gleichmäßige Beschichtung zu gewährleisten. Zum anderen ist es notwendig, das Silikonöl nur in geringen Mengen dosiert aufzubringen, um sowohl ein Verstopfen der Kanüle mit überschüssigem silikonöl während der Injektion als auch ein Vermischen des Silikonöls mit dem Wirkstoff zu vermeiden.

Aus der DE 30 45 950 ist bereits ein Verfahren und eine Vorrichtung zum Silikonisieren reibend beanspruchter Flächen von Spritzen bekannt, bei welchem das in einem Gefäß vorgesehene Silikonöl über eine schräg angeordnete, mit seinem Rand in das Silikonbad tauchende Übertragungsrolle auf die Dichtmanschette einer aus Kolben, Zylinder und Dichtmanschette bestehenden dreiteiligen Spritze übertragen wird. Dazu ist die Übertragungsrolle als flache und rotationsmetrische Scheibe ausgebildet und drehangetrieben. Auch die Dichtmanschette ist um ihre Längsachse drehbar und angetrieben, wodurch die Dichtlippen der Dichtmanschette gleichmäßig mit Silikonöl benetzt werden. Nachteilig ist hierbei jedoch, dass bereits die gesamte, für die Beschichtung erforderliche Menge an Silikonöl vorab auf die Dichtmanschette aufgebracht werden muss, so dass beim Einschieben der Dichtmanschette in den Spritzenzylinder mit zunehmender Wegstrecke mit einer Abnahme der aufgetragenen Menge an Silikonöl gerechnet werden muss.

Ferner ist es aus der DE 32 20 686 C2 bereits bekannt, das überschüssige Silikonöl, das bereits beim Auftragsvorgang unter den Beschichtungsring gelangt ist, durch eine Absaugvorrichtung zu entfernen. Dies macht jedoch zusätzlich eine Absaugleitung sowie eine Absaugvorrichtung erforderlich.

Der Erfindung liegt die Aufgabe zugrunde, ein Verfahren der eingangs genannten Art anzugeben und so zu verbessern, daß eine gleichmäßige, homogene Beschichtung erreicht und zugleich auf einfache weise sichergestellt wird, daß kein überschüssiges Silikonöl an der Innenseite der Glaszylinder haften bleibt.

Ein diese Aufgabe lösendes Verfahren ist dadurch gekennzeichnet, daß die Benetzung des Beschichtungsrings über eine im Bereich des Beschichtungsrings mündende Speiseleitung für Silikonöl erfolgt.

Der durch die Erfindung erreichte Vorteil besteht darin, daß zunächst durch den Beschichtungsring die gleichmäßige Benetzung bei der Bewegung zum kanülenseitigen Ende des Zylinders hin erreicht wird, und daß beim Zurückziehen des Beschichtungsrings der diesem nachlaufende Abstreifring dafür sorgt, daß überschüssiges silikonöl aus dem Glaszylinder wieder ausgetragen wird.

In vorteilhafter und optimierter Ausgestaltung des Verfahrens weist der gebildete Silikonfilm zweckmäßigerweise eine Dicke von etwa 2 Mikrometer auf.

In vorrichtungsmäßiger Hinsicht wird die Aufgabe gelöst durch einen Beschichtungsring sowie einen Abstreifring, die beide mit gegenseitigem axialem Abstand auf einem Silikonisierdorn angeordnet sind, wobei der Silikonisierdorn einen axial verlaufenden Kanal für die Einspeisung des Silikonöls aufweist, dessen eines Ende im Bereich des Beschichtungsrings mündet und dessen anderes Ende zum Anschluß an eine Speiseleitung eingerichtet ist.

In vorteilhafter Weiterbildung der Erfindung sind der Beschichtungsring und der Abstreifring als O-Ring ausgebildet. Dabei ist es im Rahmen der Erfindung zweckmäßig, wenn der Beschichtungsring und der Abstreifring jeweils in einer Ringnut des silikonisierdoras angeordnet sind, wobei der Kanal für die Einspeisung des Silikonöls in die Ringnut des Beschichtungsrings mündet.

Um eine gleichmäßige Verteilung des Silikonöls auf dem Beschichtungsring zu erreichen, hat es sich als günstig erwiesen, wenn in die Ringnut des Beschichtungsrings zwei oder mehrere radial verlaufende, gleichmäßig über den Umfang verteilt angeordnete Mündungskanäle eintreten.

Schließlich kann im Rahmen der Erfindung noch vorgesehen sein, daß die Ringnut für den Abstreifring breiter ausgebildet ist zur Aufnahme wenigstens eines Stützringes, der auf der dem Beschichtungsring abgewandten Seite angeordnet ist.

Im folgenden wird die Erfindung an einem in der Zeichnung dargestellten Ausführungsbeispiel näher erläutert; es zeigen:
- Fig. 1: eine vollständige, wenngleich teilweise nur schematische Darstellung der Vorrichtung zum Beschichten von Spritzenzylindern,

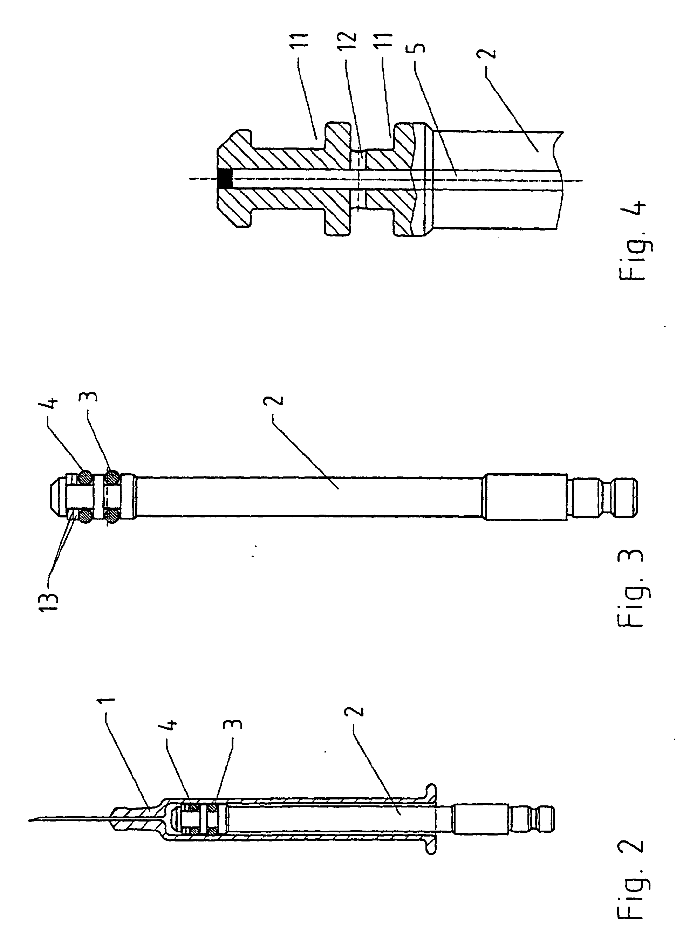

- Fig. 2: eine Detaildaratellung aus Fig. 1, nämlich einen Spritzenzylinder mit eingefahrenem Silikonisierdorn,
- Fig. 3: eine Detailansicht des Silikonisierdorns,
- Fig. 4: den Gegenstand nach Fig. 3, jedoch ohne Beschichtungs- und Abstreifring, sowie teilweise im Schnitt dargestellt.

Die in der Zeichnung dargestellte Vorrichtung dient zur Beschichtung von Primärpackmitteln, insbesondere zur innenseitigen Beschichtung von kleinvolumigen Spritzenzylindern 1 mit einem Silikonfilm.

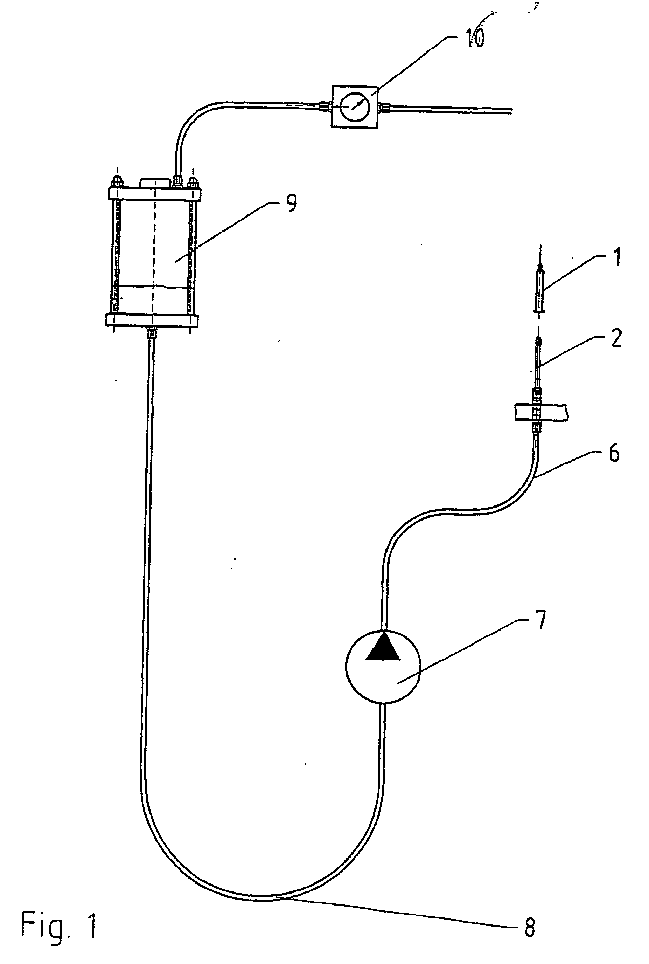

Dazu weist die Vorrichtung einen Silikonisierdorn 2 auf, auf welchem ein Beschichtungsring 3 sowie ein Abstreifring 4 mit gegenseitigem axialen Abstand angeordnet sind. Der silikonisierdorn 2 weist einen axial verlaufenden Kanal 5 für die Einspeisung des Silikonöls auf, wobei das eine Ende dieses Kanals 5 im Bereich des Beschichtungsringes 3 mündet und das andere Ende zum Anschluß an eine Speiseleitung 6 eingerichtet ist. Diese Speiseleitung 6 führt, wie aus Fig. 1 zu ersehen, zu einer Pumpe 7, die über einen weiteren Schlauch 8 mit einem Silikonvorratsbehälter 9 in Verbindung steht. Dieser Silikonvorratsbehälter 9 kann zusätzlich über ein Druck-Regelventil 10 beaufschlagt werden.

In der in Fig. 1 dargestellten Ausgangsposition wird zunächst der Beschichtungsring 3 mit Silikonöl benetzt, worauf der Silikonisierdorn 2 über eine in der Zeichnung nicht näher dargestellte Hubvorrichtung in den Spritzenzylinder 1 eingebracht und der Beschichtungsring 3 in axialer Richtung an der Innenwand des Spritzenzylinders 1 entlang geführt wird. Der Beschichtungsring 3 liegt hierbei der Innenwand vollständig an, so daß eine schon weitgehend gleichmäßige und vollständige Beschichtung der Innenwand mit Silikonöl erfolgt.

Im Anschluß daran wird der Silikonisierdorn 2 wieder zurückgezogen, worauf dieser in Bewegungsrichtung hinter dem Beschichtungsring 3 angeordnete Abatreifring 4 das überschüssige Silikonöl unter Bildung eines homogenen, gleichmäßig dicken Silikonfilms abträgt. Die Gestaltung des Abstreifringes 4 sowie dessen Anpreßdruck sind dabei so eingestellt, daß der verbleibende Silikonfilm eine Dicke von etwa 2 Mikrometer aufweist.

Der Beschichtungsring 3 und der Abstreifring 4 sind, wie insbesondere die Fig. 3 erkennen läßt, als O-Ring ausgebildet. Weiter sind der Beschichtungsring 3 und der Abstreifring 4 jeweils in einer Ringnut 11 des Silikonisierdorns 2 angeordnet, wobei der Kanal 5 für die Einspeisung des Silikonöls in die Ringnut 11 des Beschichtungsrings 3 mündet.

Um eine möglichst gleichmäßige Benetzung des Beschichtungsrings 3 zu erreichen, treten in die Ringnut 11 des Beschichtungsringes 3 zwei radial verlaufende Mündungskanäle 12 ein. Es ist jedoch ebenso denkbar, auch mehrere, gleichmäßig über den Umfang verteilt angeordnete Mündungskanäle 12 vorzusehen.

Wie aus der Fig. 3 hervorgeht, ist die Ringnut 11 für den Abstreifring 4 breiter ausgebildet und dient zusätzlich der Aufnahme von zwei Stützringen 13, die auf der dem Beschichtungsring 3 abgewandten Seite angeordnet sind.

## Patentansprüche

1. Verfahren zur Beschichtung von Primärpackmitteln, insbesondere zur innenseitigen Beschichtung von kleinvolumigen Spritzenzylindern (1) mit einem Silikonfilm, bei welchem zunächst ein Beschichtungsring (3) mit Silikonöl benetzt wird, daß sodann der Beschichtungering (3) in den Spritzenzylinder (1) eingebracht und in axialer Richtung an der Innenwand entlang geführt wird, wobei der Beschichtungsring (3) der Innenwand vollständig anliegt, und daß schließlich beim Zurückziehen des Beschichtungsrings (3) ein in Bewegungsrichtung dahinter angeordneter Abstreifring (4) das überschüssige Silikonöl unter Bildung eines homogenen, gleichmäßig dicken Silikonfilms abträgt, **dadurch gekennzeichnet, daß** die Benetzung des Beschichtungsrings (3) über eine im Bereich des Beschichtungsrings (3) mündende Speiseleitung (5,6,12) für Silikonöl erfolgt.

2. Verfahren nach Anspruch 1, **dadurch gekennzeichnet, daß** der gebildete Silikonfilm eine Dicke von etwa 2 Mikrometer aufweist.

3. Vorrichtung zur Durchführung des Verfahrens nach den Ansprüchen 1 oder 2, **gekennzeichnet durch** einen Beschichtungsring (3) sowie einen Abetreifring (4), die beide mit gegenseitigem axialem Abstand auf einem Silikonisierdorn (2) angeordnet sind, wobei der Silikonisierdorn (2) einen axial verlaufenden Kanal (5) für die Einspeisung des Silikonöls aufweist, dessen eines Ende im Bereich des Beschichtungsrings (3) mündet und dessen anderes Ende zum Anschluß an eine Speiseleitung (6) eingerichtet ist.

4. Vorrichtung nach Anspruch 3, **dadurch gekennzeichnet, daß** der Beschichtungsring (3) und der Abstreifring (4) als O-Ring ausgebildet sind.

5. Vorrichtung nach Anspruch 3 oder 4, **dadurch gekennzeichnet, daß** der Beschichtungsring (3) und der Abstreifring (4) jeweils in einer Ringnut (11) des Silikonieierdorns (2) angeordnet sind, wobei der Kanal (5) für die Einspeisung des Silikonöls in die Ringnut (11) des Beschichtungsringe (3) mündet.

6. Vorrichtung nach Anspruch 5, **dadurch gekennzeichnet, daß** in die Ringnut (11) des Beschichtungsrings (3) zwei oder mehrere radial verlaufende, gleichmäßig über den Umfang verteilt angeordnete Mündungskanäle (12) eintreten.

7. Vorrichtung nach Anspruch 5 oder 6, **dadurch gekennzeichnet, daß** die Ringnut (11) für den Abstreifring (4) breiter ausgebildet ist zur Aufnahme wenigstens eines Stützrings (13), der auf der dem Beschichtungsring (3) abgewandten Seite angeordnet ist.

## Claims

1. A process for coating primary packaging means, in particular for internally coating small-volume syringe cylinders (1) with a silicone film, in which firstly a coating ring (3) is wetted with silicone oil, then the coating ring (3) is introduced into the syringe cylinder (1) and passed in the axial direction along the inside wall, wherein the coating ring (3) bears completely against the inside wall, and finally upon retraction of the coating ring (3) a scraper ring (4) arranged therebehind in the direction of movement removes the excess silicone oil, forming a homogenous silicone film of uniform thickness, **characterised in that** wetting of the coating ring (3) is effected by way of a feed conduit (5, 6, 12) for silicone oil, which opens in the region of the coating ring (3).

2. A process according to claim 1 **characterised in that** the silicone film formed is of a thickness of about 2 micrometers.

3. Apparatus for carrying out the process according to claims 1 or 2 **characterised by** a coating ring (3) and a scraper ring (4) which are both arranged at a mutual axial spacing on a siliconisation rod (2), wherein the siliconisation rod (2) has an axially extending duct (5) for the feed of the silicone oil, of which one end opens in the region of the coating ring (3) and the other end is adapted for connection to a feed conduit (6).

4. Apparatus according to claim 3 **characterised in that** the coating ring (3) and the scraper ring (4) are in the form of an O-ring.

5. Apparatus according to claim 3 or claim 4 **characterised in that** the coating ring (3) and the scraper ring (4) are respectively arranged in an annular groove (11) of the siliconisation rod (2), wherein the duct (5) for the feed of the silicone oil opens into the annular groove (11) of the coating ring (3).

6. Apparatus according to claim 5 **characterised in that** two or more radially extending communication ducts (12) arranged distributed uniformly over the periphery pass into the annular groove (11) of the coating ring (3).

7. Apparatus according to claim 5 or claim 6 **characterised in that** the annular groove (11) for the scraper ring (4) is wider for accommodating at least one support ring (13) which is arranged on the side remote from the coating ring (3).

## Revendications

1. Procédé pour revêtir des moyens d'emballage primaire, notamment pour revêtir l'intérieur de cylindres de seringue (1) de petit volume par un film de silicone, dans lequel :
- tout d'abord une bague de revêtement (3) est mouillée par de l'huile de silicone,
- ensuite la bague (3) est introduite dans le cylindre de seringue (1) et déplacée en direction axiale le long de la paroi interne en étant totalement en contact avec celle-ci,
- enfin, en tirant en arrière la bague de revêtement (3), une bague de raclage (4) située derrière celle-ci dans ce mouvement en arrière, retire l'huile de silicone en excès avec formation d'un film de silicone homogène et d'épaisseur uniforme,
**caractérisé en ce que** le mouillage de la bague de revêtement (3) est assuré par l'intermédiaire d'une conduite d'alimentation (5, 6, 12) débouchant dans la zone de la bague de revêtement (3).

2. Procédé selon la revendication 1, **caractérisé en ce que** le film de silicone formé a une épaisseur d'environ 2 microns.

3. Dispositif pour la mise en oeuvre du procédé selon la revendication 1 ou 2, **caractérisé en ce qu'**il comprend une bague de revêtement (3) et une bague de raclage (2) montées avec espacement axial sur une broche de siloconation (2) qui présente, pour l'alimentation en huile de silicone, un canal axial (5) dont une extrémité débouche dans la zone de la bague de revêtement (3), tandis que l'autre extrémité est conçue pour être raccordée à une conduite d'alimentation (6).

4. Dispositif selon la revendication 1, **caractérisé en ce que** la bague de revêtement (3) et la bague de raclage (4) sont des bagues O-Ring.

5. Dispositif selon la revendication 3 ou 4, **caractérisé en ce que** la bague de revêtement (3) et la bague de raclage (4) sont disposées chacune dans une gorge annulaire (11) de la broche de siliconation (2), le canal (5) assurant l'alimentation en huile de silicone débouchant dans la gorge annulaire (11) de la bague de revêtement (3).

6. Dispositif selon la revendication 5, **caractérisé en ce que** dans la gorge annulaire (11) de la bague de revêtement (3) débouchent deux canaux, ou plus, orientés radialement et répartis régulièrement en périphérie.

7. Dispositif selon la revendication 5 ou 6, **caractérisé en ce que** la gorge annulaire (11) pour la bague de raclage (4) est plus large afin d'accueillir au moins une bague de soutien (13) disposée du côté éloigné de la bague de revêtement (3).
